(19) Europäisches Patentamt
European Patent Office
Office européen des brevets

(11) **EP 4 098 361 A1**

(12) **EUROPEAN PATENT APPLICATION**
published in accordance with Art. 153(4) EPC

(43) Date of publication:
**07.12.2022 Bulletin 2022/49**

(21) Application number: **20916925.9**

(22) Date of filing: **01.10.2020**

(51) International Patent Classification (IPC):
**B01J 37/00** *(2006.01)*　　**B01J 23/887** *(2006.01)*
**C07B 61/00** *(2006.01)*　　**C07C 51/235** *(2006.01)*
**C07C 57/055** *(2006.01)*

(52) Cooperative Patent Classification (CPC):
**B01J 23/887; B01J 37/00; C07B 61/00;
C07C 51/235; C07C 57/04;** Y02P 20/584

(86) International application number:
**PCT/JP2020/037422**

(87) International publication number:
**WO 2021/152916 (05.08.2021 Gazette 2021/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.01.2020 JP 2020014788**

(71) Applicant: **Mitsubishi Chemical Corporation
Chiyoda-ku
Tokyo 100-8251 (JP)**

(72) Inventors:
• **ABE, Yoshimune**
**Tokyo 100-8251 (JP)**
• **KANUKA, Nariyasu**
**Tokyo 100-8251 (JP)**
• **OKADA, Shigeki**
**Tokyo 100-8251 (JP)**
• **KOUNO, Souhei**
**Tokyo 100-8251 (JP)**

(74) Representative: **Vossius & Partner
Patentanwälte Rechtsanwälte mbB
Siebertstraße 3
81675 München (DE)**

(54) **METHOD FOR PRODUCING CATALYST, AND METHOD FOR PRODUCING ACRYLIC ACID**

(57)　Provided is a method for producing a catalyst having a high raw material conversion rate and a high product selectivity as well as excellent yield of unsaturated carboxylic acid, the catalyst being used in a vapor-phase catalytic oxidation reaction for producing an unsaturated carboxylic acid such as acrylic acid or methacrolein from an unsaturated aldehyde such as acrolein or methacrolein. A method for producing a catalyst for synthesizing an unsaturated carboxylic acid, the method including a molding process of molding a powder containing each catalyst component element to produce a catalyst precursor, wherein a sulfur-containing inorganic compound is added to the powder, and the powder is molded in the molding process.

**Description**

Technical Field

The present invention relates to a method for producing a catalyst used for producing unsaturated carboxylic acids such as acrylic acid and methacrylic acid by vapor-phase catalytic oxidation of unsaturated aldehydes such as acrolein and methacrolein.

The present invention also relates to a method for producing acrylic acid using this catalyst.

Background Art

It is well known that a catalyst containing molybdenum and vanadium is useful as a catalyst used for producing unsaturated carboxylic acids such as acrylic acid and methacrylic acid by vapor-phase catalytic oxidation of unsaturated aldehydes such as acrolein and methacrolein. The catalyst has been widely applied to industrial use.

PTL 1 and the like are known as prior art documents relating to a method for producing a catalyst containing molybdenum and vanadium for synthesizing an unsaturated carboxylic acid.

In PTL 1, a supported catalyst is produced by a method in which a catalytically active component and / or a precursor thereof containing a liquid binder component, molybdenum and vanadium are supplied to a tumbling granulator, and granulated at a specific relative centrifugal acceleration.

PTL 1: JP2015-96497 A

The catalyst for synthesizing unsaturated carboxylic acid obtained by the known method does not satisfy both of excellent raw material conversion rate and product selectivity. Therefore, when the unsaturated carboxylic acid is produced using the catalyst, the yield of the unsaturated carboxylic acid is not satisfactory.

Summary of Invention

It is an object of the present invention to provide a catalyst having a high raw material conversion rate and a high product selectivity as well as excellent yield of unsaturated carboxylic acid, the catalyst being used in a vapor-phase catalytic oxidation reaction for producing an unsaturated carboxylic acid such as acrylic acid or methacrylic acid from an unsaturated aldehyde such as acrolein or methacrolein.

The present inventors have found that the above object can be achieved by using a specific molding aid.

The gist of the present invention is as follows. Summary of the present invention is as follows.

[1] A method for producing a catalyst for synthesizing an unsaturated carboxylic acid, the method comprising a molding process of molding a powder containing each catalyst component element to produce a catalyst precursor, wherein a sulfur-containing inorganic compound is added to the powder, and the powder is molded in the molding process.

[2] The method for producing a catalyst according to [1], wherein the sulfur-containing inorganic compound does not contain the catalyst component element.

[3] The method for producing a catalyst according to [1] or [2], wherein the sulfur-containing inorganic compound is a sulfate.

[4] The method for producing a catalyst according to [3], wherein the sulfate is ammonium sulfate.

[5] Production of the catalyst according to any one of [1] to [4], wherein an amount of the sulfur-containing inorganic compound added is 1.0 part by weight or more and 15 parts by weight or less with respect to 100 parts by weight of the powder.

[6] The method for producing a catalyst according to any one of [1] to [5], wherein an organic compound is further added to the powder, and the powder is molded in the molding process.

[7] The method for producing a catalyst according to [6], wherein the organic compound is glycerin.

[8] The method for producing a catalyst according to [6] or [7], wherein an amount of the organic compound added is 0.3 parts by weight or more and 15 parts by weight or less with respect to 100 parts by weight of the powder.

[9] The method for producing a catalyst according to any one of [1] to [8], wherein the catalyst component element contains molybdenum and vanadium.

[10] The method for producing a catalyst according to any one of [1] to [9], wherein the molding process is a process of supporting the powder on a surface of a carrier and granulating the powder into a catalyst precursor.

[11] A method for producing acrylic acid in which acrolein is oxidized by a vapor-phase catalytic oxidization reaction with an oxygen-containing gas in the presence of a catalyst produced by the catalyst production method according to any one of [1] to [10].

Advantageous Effects of Invention

According to the present invention, a catalyst is provided, the catalyst being used in a vapor-phase catalytic oxidation reaction for synthesizing an unsaturated carboxylic acid such as acrylic acid or methacrylic acid from an unsaturated aldehyde such as acrolein or methacrolein, and the catalyst having a high raw material conversion rate and product selectivity as well as excellent yield of unsaturated carboxylic acid.

Description of Embodiments

Hereinafter, embodiments of the method for producing the unsaturated carboxylic acid synthesis catalyst of the present invention (hereinafter, sometimes referred to as "catalyst of the present invention") will be described in detail.
The catalyst of the present invention is a catalyst for synthesizing an unsaturated carboxylic acid such as acrylic acid or methacrylic acid by vapor-phase catalytic oxidation using an unsaturated aldehyde such as acrolein or methacrolein as a raw material with an oxygen-containing gas.

[Producing Method of Catalyst]

The method for producing a catalyst of the present invention includes a molding process in which a powder (hereinafter, the powder may be referred to as "raw material powder") containing an element constituting the catalyst (hereinafter, the element may be referred to as a "catalyst component element") is molded to be a catalyst precursor, and the method is characterized in that a specific molding aid is added in the molding process.
The raw material powder can be obtained, for example, through the following process.
A compound including a catalyst component element is used as a compound serving as a catalyst supply source (hereinafter, referred to as "source compound"). Each source compound including the catalyst component element is added to a solvent or a solution to be integrated, and then the solvent or solution is heated so as to obtain a prepared liquid (liquid preparation process). Next, the prepared liquid is dried to obtain a raw material powder (drying process). The raw material powder is molded to obtain a catalyst precursor of the catalyst, and then the precursor is fired (firing process) to obtain the catalyst of the present invention.

[Liquid Preparation Process]

The liquid preparation process is a process in which each source compound is preferably integrated in an aqueous system and heated to obtain a prepared liquid.
The integration in the aqueous system means that each source compound is added to an aqueous solvent or solution to perform integration. The aqueous solvent is an aqueous medium for dissolving or suspending each source compound. This aqueous solvent is a solvent consisting of water, an organic solvent compatible with water such as methanol and ethanol, or a mixture thereof. The aqueous solution is a solution in which one or more source compounds are dissolved, suspended or integrated in an aqueous solvent.
The above-mentioned integration means that the aqueous solution or the aqueous dispersion of each source compound is mixed all at once or stepwise, and heated as necessary. The integration includes specifically, a method of collectively mixing each source compound; a method of collectively mixing each source compound and then heating; a method of stepwise mixing of each source compound; a method of repeating stepwise mixing each source compound and then heating; and a method of combining these methods. All of these methods are included in the concept of integration of each source compound.
The above-mentioned heating means that the mixed liquid or the mixed dispersion obtained by the above-mentioned integration is stirred at a predetermined temperature for a predetermined time. This heating increases the viscosity of the mixed solution or the mixed dispersion. This is effective in alleviating sedimentation of the solid component in the mixed dispersion liquid, and particularly in suppressing non-uniformity of the component in the next drying process. As a result, the catalytic activity such as the raw material conversion rate and the product selectivity of the obtained final product catalyst becomes better.
The temperature in the heating is preferably 60 °C to 100 °C, more preferably 60 °C to 90 °C, still more preferably 70 °C to 85 °C. When the heating temperature is 60 °C or higher, the effect of the heat treatment is sufficient, and good catalytic activity tends to be obtained. When the heating temperature is 100 °C or lower, a solution tank does not require a pressure-resistant container, handling is simple, and it is preferable in terms of economy and operability. When the heating temperature is 90 °C or lower, evaporation of water during the heat treatment is suppressed, and industrial application tends to be advantageous.
The heating time is preferably 2 hours to 12 hours, more preferably 3 hours to 8 hours. When the heating time is 2 hours or more, the activity and selectivity of the catalyst tend to be sufficiently expressed. Even if the heating time is excessively long, almost no improvement in the heating effect can be obtained. Therefore, the heating time is preferably 12 hours or less.

Any method can be adopted as the stirring method. Examples of the stirring method include a method using a stirrer having a stirring blade; a method using an external circulation by a pump; and the like.

[Source Compound]

The catalyst of the present invention preferably contains molybdenum (Mo) and vanadium (V) as catalyst component elements, and it is more preferable that the catalyst of the present invention contains copper (Cu) as the other catalyst component element. The catalyst of the present invention may contain further one or more components such as antimony (Sb), silicon (Si), carbon (C), niobium (Nb), tungsten (W), magnesium (Mg), calcium (Ca), strontium (Sr), barium (Ba), zinc (Zn), iron (Fe), cobalt (Co), nickel (Ni), and bismuth (Bi).

Examples of the source compound of molybdenum (Mo) include ammonium paramolybdate, molybdenum trioxide, molybdenum acid, ammonium phosphomolybdate, and phosphomolybdic acid. One of these may be used alone, or two or more thereof may be mixed and used.

Examples of the source compound of vanadium (V) include ammonium vanadate, ammonium metavanadate, vanadium pentoxide, vanadium oxalate, vanadium sulfate and the like. One of these may be used alone, or two or more thereof may be mixed and used.

It is preferable to add vanadium so that the atomic number ratio of the catalyst component element is more than 0 and 12 or less when the molybdenum atom is expressed as 12. The atomic number ratio of the catalyst component element of vanadium is more preferably 0.1 or more and 6 or less, still more preferably 0.5 or more and 5 or less, and particularly preferably 1 or more and 3 or less when the molybdenum atom is expressed as 12. When the amount of vanadium added is within this range, it is possible to obtain a catalyst having an excellent raw material conversion rate and capable of producing an unsaturated carboxylic acid with a high selectivity.

Examples of the source compound of niobium (Nb) include niobium hydroxide. One of these may be used alone, or two or more thereof may be mixed and used.

Examples of the source compound of tungsten (W) include tungstic acid or a salt thereof. One of these may be used alone, or two or more thereof may be mixed and used.

When at least one element selected from niobium and tungsten is used, the element is added so that the total atomic number ratio of atoms of the catalyst component elements is preferably 0 or more and 12 or less, more preferably 0.1 or more and 6 or less, and further preferably 0.5 or more and 4 or less, when the molybdenum atom is expressed as 12. When the amount of the element added is within this range, it is possible to obtain a catalyst having an excellent raw material conversion rate and capable of producing an unsaturated carboxylic acid with a high selectivity.

Examples of the copper (Cu) source compound include copper sulfate, copper nitrate, and cuprous chloride. One of these may be used alone, or two or more thereof may be mixed and used.

Copper is preferably added so that the atomic number ratio of the catalyst component element is more than 0 and 12 or less, more preferably 0.1 or more and 6 or less, and further preferably 0.5 or more and 4 or less, when the molybdenum atom is expressed as 12. When the amount of copper added is within this range, it is possible to obtain a catalyst having an excellent raw material conversion rate and capable of producing an unsaturated carboxylic acid with a high selectivity.

Examples of the source compound of magnesium (Mg) include magnesium oxide, magnesium carbonate, magnesium sulfate and the like. One of these may be used alone, or two or more thereof may be mixed and used.

Examples of the calcium (Ca) source compound include calcium oxide, calcium carbonate, calcium hydroxide and the like. One of these may be used alone, or two or more thereof may be mixed and used. One of these may be used alone, or two or more thereof may be mixed and used.

Examples of the source compound of strontium (Sr) include strontium oxide, strontium carbonate, strontium hydroxide, strontium nitrate and the like. One of these may be used alone, or two or more thereof may be mixed and used.

Examples of the source compound of barium (Ba) include barium oxide, barium carbonate, barium nitrate, barium acetate, barium sulfate and the like. One of these may be used alone, or two or more thereof may be mixed and used.

Examples of the source compound of zinc (Zn) include zinc oxide, zinc carbonate, zinc hydroxide, zinc nitrate and the like. One of these may be used alone, or two or more thereof may be mixed and used.

When at least one element selected from the group consisting of magnesium, calcium, strontium, barium and zinc is used, these elements are added so that the total atomic number ratio of the catalyst component elements is preferably 0 or more and 8 less, more preferably 0 or more and 6 or less, still more preferably 0 or more and 4 or less when the molybdenum atom is expressed as 12. When the amount of these elements added is within this range, it is possible to obtain a catalyst having an excellent raw material conversion rate and capable of producing an unsaturated carboxylic acid with a high selectivity.

Examples of the source compound of antimony (Sb) include antimony oxide such as antimony trioxide and antimony pentoxide, trivalent antimony compounds such as antimony acetate, and pentavalent antimony compounds. One of these may be used alone, or two or more thereof may be mixed and used.

When antimony is used, it is preferable to add antimony so that the atomic number ratio of the catalyst component element is 0 or more and 500 or less, more preferably 0.1 or more and 100 or less, and further preferably 0.2 or more and 50 or less, when the molybdenum atom is expressed as 12. When the amount of antimony added is within this range, it is possible to obtain a catalyst having an excellent raw material conversion rate and capable of producing an unsaturated carboxylic acid with a high selectivity.

Examples of the source compound of iron (Fe) include ferric nitrate, ferric sulfate, ferric chloride, ferric acetate and the like. One of these may be used alone, or two or more thereof may be mixed and used.

Examples of the source compound of cobalt (Co) include cobalt nitrate, cobalt sulfate, cobalt chloride, cobalt carbonate, cobalt acetate and the like. One of these may be used alone, or two or more thereof may be mixed and used.

Examples of the source compound of nickel (Ni) include nickel nitrate, nickel sulfate, nickel chloride, nickel carbonate, nickel acetate and the like. One of these may be used alone, or two or more thereof may be mixed and used.

Examples of the source compound of bismuth (Bi) include bismuth chloride, bismuth nitrate, bismuth oxide, and bismuth subcarbonate. One of these may be used alone, or two or more thereof may be mixed and used.

When at least one element selected from the group consisting of iron, cobalt, nickel and bismuth is used, these elements are added so that the total atomic number ratio of the catalyst component elements is preferably 0 or more and 500 or less, more preferably 0 or more and 400 or less, still more preferably 0 or more and 300 or less, when the molybdenum atom is expressed as 12. When the amount of these elements added is within this range, it is possible to obtain a catalyst having an excellent raw material conversion rate and capable of producing an unsaturated carboxylic acid with a high selectivity.

Examples of the source compound of silicon (Si) include silica, granular silica, colloidal silica, and fumed silica. One of these may be used alone, or two or more thereof may be mixed and used.

Examples of the source compound of silicon (Si) and carbon (C) include green silicon carbide and black silicon carbide. One of these may be used alone, or two or more thereof may be mixed and used.

When silicon is used, silicon is added so that the atomic number ratio of the catalyst component element is preferably 0 or more and 500 or less, more preferably 0 or more and 400 or less, still more preferably 0 or more and 300 or less, when the molybdenum atom is expressed as 12. When the amount of silicon added is within this range, it is possible to obtain a catalyst having an excellent raw material conversion rate and capable of producing an unsaturated carboxylic acid with a high selectivity.

When carbon is used, carbon is added so that the atomic number ratio of the catalyst component element is preferably 0 or more and 500 or less, more preferably 0 or more and 400 or less, still more preferably 0 or more and 300 or less, when the molybdenum atom is expressed as 12. When the amount of carbon added is within this range, it is possible to obtain a catalyst having an excellent raw material conversion rate and capable of producing an unsaturated carboxylic acid with a high selectivity.

[Method of Adding Source Compound]

The liquid preparation process may be any of the following.

All of each source compound is made into one preparation liquid.

Each source compound is used alone or divided into several groups to prepare multiple preparation liquids. Then, the plurality of preparation liquids are mixed at once or in order to form one preparation liquid.

One or more preparation liquids are dried and then fired to produce a solid. The solid is then added to the preparation liquid containing the remaining source compounds to prepare a new preparation liquid.

[Drying Process]

The raw material powder can be obtained by drying the obtained preparation liquid in a drying process.

There is no particular limitation on the drying treatment method in the drying process. Examples of the drying treatment method include a method using a normal spray dryer, slurry dryer, drum dryer and the like.

The raw material powder obtained by the drying treatment may be further heat-treated, if necessary. The heat treatment is a treatment performed in the air in a temperature range of 200 °C to 400 °C, preferably 250 °C to 350 °C for a short time. The heating method is not particularly limited, but the method is for example a method of heating the powder in a fixed state using a normal box-type heating furnace, a tunnel-type heating furnace, or the like, or a method of heating while flowing the powder using a rotary kiln or the like.

The dried product obtained by the drying treatment may be further subjected to a treatment such as pulverization, and the powder thus obtained is also included in the raw material powder in the present invention.

The raw material powder obtained by the drying process preferably contains molybdenum and vanadium, and more preferably copper further. Among them, the raw material powder is preferably a raw material powder having a composition represented by the following general formula (1).

$$Mo_{12}V_aX_bCu_cY_dSb_eZ_fSi_gC_hO_i \qquad (1)$$

(In the formula (1), X represents Nb and / or W. Y represents at least one element selected from the group consisting of Mg, Ca, Sr, Ba and Zn. Z represents at least one element selected from the group consisting of Fe, Co, Ni and Bi. The a to i indicate the atomic ratio of each element. $0 < a \leq 12$, $0 \leq b \leq 12$, $0 < c \leq 12$, $0 \leq d \leq 8$, $0 \leq e \leq 500$, $0 \leq f \leq 500$, $0 \leq g \leq 500$, $0 \leq h \leq 500$. The i is a value that satisfies the oxidation state of other elements.)

[Molding Aid]

In the method for producing a catalyst of the present invention, a sulfur-containing inorganic compound is added to the raw material powder as a specific molding aid in the molding process described later.

The sulfur-containing inorganic compound used in the present invention may be any sulfur-containing inorganic compound, and is not particularly limited. It is preferable that the sulfur-containing inorganic compound used in the present invention does not contain a catalyst component element because the composition of the catalyst can be easily controlled to the desired suitable composition.

As the sulfur-containing inorganic compound, at least one selected from the group consisting of sulfate, sulfite, thiosulfate and sulfamic acid is preferable from the viewpoint of obtaining a catalyst having a high raw material conversion rate and a good product selectivity. As the sulfur-containing inorganic compound, sulfate is particularly preferable.

The sulfur-containing inorganic compound is preferably at least one selected from the group consisting of sodium-containing compounds, potassium-containing compounds and ammonium-containing compounds from the viewpoint of obtaining a catalyst having a high raw material conversion rate and a good product selectivity. As the sulfur-containing inorganic compound, an ammonium-containing compound is particularly preferable.

Examples of the sulfate include sodium sulfate, potassium sulfate, ammonium sulfate and the like. As the sulfate, ammonium sulfate is particularly preferable.

Examples of sulfite include sodium sulfite, potassium sulfite, and ammonium sulfite. As the sulfite, ammonium sulfite is particularly preferable.

Examples of thiosulfate include sodium thiosulfate, potassium thiosulfate, and ammonium thiosulfate. As the thiosulfate, ammonium thiosulfate is particularly preferable.

Examples of sulfamic acid include sodium sulfamate, potassium sulfamate, and ammonium sulfamate. As the sulfamic acid, ammonium sulfamate is particularly preferable.

These may be used alone or in combination of two or more.

Among these, sulfate is particularly preferable as the sulfur-containing inorganic compound from the viewpoint of obtaining a catalyst having a high raw material conversion rate and a good product selectivity. Ammonium sulfate is preferable as the sulfate because it can be easily decomposed during firing.

The amount of the sulfur-containing inorganic compound added is preferably 1.0 part by weight or more and 15 parts by weight or less with respect to 100 parts by weight of the raw material powder. The lower limit of the amount of the sulfur-containing inorganic compound added is more preferably 2 parts by weight, still more preferably 3 parts by weight, and particularly preferably 4 parts by weight. The upper limit of the amount of the sulfur-containing inorganic compound added is more preferably 14 parts by weight, still more preferably 13 parts by weight, and particularly preferably 12 parts by weight. When the amount of the sulfur-containing inorganic compound added is within the above range, it is possible to surely obtain a catalyst having a high raw material conversion rate and a good product selectivity.

The form when the sulfur-containing inorganic compound is added to the raw material powder is preferably an aqueous solution. In this case, the concentration of the sulfur-containing inorganic compound in the sulfur-containing inorganic compound aqueous solution is preferably 5% by weight or more and 40% by weight or less. The lower limit of the concentration of the sulfur-containing inorganic compound is more preferably 10% by weight, and the upper limit is more preferably 35% by weight. When the concentration of the sulfur-containing inorganic compound is in the above range, it becomes easy to uniformly add and disperse the sulfur-containing inorganic compound to the raw material powder.

In the present invention, it is preferable to add further an organic compound as a molding aid to the raw material powder in the molding process from the viewpoint of obtaining a catalyst having a high strength, a high raw material conversion rate and a good product selectivity.

Examples of the organic compound include ethylene glycol, glycerin, propionic acid, maleic acid, benzyl alcohol, propyl alcohol, butyl alcohol, cellulose, methyl cellulose, starch, polyvinyl alcohol, stearic acid and phenol. One of these may be used alone, or two or more thereof may be mixed and used.

Among these organic compounds, glycerin is preferable because an excellent catalyst can be obtained in terms of raw material conversion rate and product selectivity by adding it to the raw material powder together with the sulfur-containing inorganic compound.

When an organic compound is used, the amount added is preferably 0.3 parts by weight or more and 15 parts by weight or less with respect to 100 parts by weight of the raw material powder. The lower limit of the amount of the organic compound added is more preferably 0.4 parts by weight, further preferably 0.5 parts by weight, particularly preferably 0.6 parts by weight, and the upper limit is more preferably 13 parts by weight, still more preferably 11 parts by weight, particularly preferably 9 parts by weight. When the amount of the organic compound added is within the above range, it is possible to obtain a catalyst having a high raw material conversion rate and a good product selectivity.

The form of the organic compound to be added is preferably an aqueous solution. In this case, the concentration of the organic compound in the aqueous solution of the organic compound is preferably 2% by weight or more and 40% by weight or less. The lower limit of the organic compound concentration is more preferably 3% by weight, and the upper limit is more preferably 30% by weight. When the concentration of the organic compound is in the above range, the organic compound can be uniformly added to the raw material powder and dispersed therein.

In the method for producing a catalyst of the present invention, a molding aid other than the sulfur-containing inorganic compound and the organic compound may be added in the molding process of the raw material powder. Examples of the molding aid other than the sulfur-containing inorganic compound and the organic compound include silica, alumina, glass flakes (scaly glass), glass fiber, silicon carbide, silicon nitride, graphite and the like. As these other molding aids, only one kind may be used, or two or more kinds may be mixed and used.

By adding these other molding aids, the strength of the obtained catalyst can be improved.

When these other molding aids are used, the addition amount thereof is preferably 20 parts by weight or less, more preferably 0.5 parts by weight or more and 20 parts by weight or less with respect to 100 parts by weight of the raw material powder.

[Molding Process]

The molding process is a process of adding a molding aid to the raw material powder obtained from the drying process and then molding the raw material powder to obtain a catalyst precursor.

The method for molding the raw material powder may be any previously known method. Examples of the method for molding the raw material powder include the following two methods (1) and (2).

(1) A method for molding a catalyst precursor by flowing a carrier that is inert to the unsaturated carboxylic acid synthesis reaction, and supplying the raw material powder to the flowing carrier, so that the raw material powder is supported on the surface of the carrier and granulated. (Hereinafter, this method may be referred to as "tumbling granulation method".)

(2) A method in which the raw material powder is supplied in a mold and pressure is applied mechanically to granulate and mold the raw material powder into a catalyst precursor. (Hereinafter, this method may be referred to as "tablet molding method".)

Among these, the tumbling granulation method is preferable because it is easy to obtain a catalyst having an excellent yield of unsaturated carboxylic acid.

The carrier used in the tumbling granulation method may be made of silica, silicon carbide, alumina, mullite, alundum, etc., and has a spherical or substantially spherical shape having a diameter or major axis diameter of preferably 2.5 mm to 10 mm, more preferably 2.5 mm to 6 mm. One of these may be used alone, or two or more thereof may be mixed and used.

A porosity of the carrier is preferably 20% to 60%, more preferably 30% to 57%, and even more preferably 40% to 55%.

A water absorption rate of the carrier is preferably 10% to 60%, more preferably 12% to 50%, and further preferably 15% to 40%.

By setting the porosity and the water absorption rate of the carrier within the above ranges, the raw material powder can be easily supported on the carrier.

A support ratio on the carrier (ratio of the total weight of the supported raw material powder and the molding aid to the weight of the carrier) is preferably 10% by weight or more and 90% by weight or less, and more preferably 20% by weight or more and 70% by weight or less.

In the tumbling granulation method, a granulator is used for example. The granulator includes a fixed container having a flat or uneven disk at the bottom thereof. The disk is rotated at a high speed to stir vigorously the carrier in the container by rotating and revolving the carrier repeatedly in the container. The raw material powder and the molding aid are fed therein and supported on the carrier.

Examples of the method for adding the molding aid in the tumbling granulation method include the following methods (1-1) to (1-5). These methods may be performed in combination of two or more.

(1-1) A method in which a raw material powder and a molding aid are mixed to prepare a homogeneous mixture, and the homogeneous mixture is supplied into the granulator and stirred.
(1-2) A method in which a raw material powder and a molding aid are simultaneously supplied into the granulator and stirred.
(1-3) A method in which while a raw material powder is stirred in the granulator, a molding aid is added to the granulator, and the powder is further stirred.
(1-4) A method in which a molding aid is added to a raw material powder to prepare a heterogeneous mixture, and the heterogeneous mixture is supplied into the granulator and stirred.
(1-5) A method in which the raw material powder and a molding aid are separately supplied into the granulator at the same time, alternately or in no particular order and stirred.

In the method (1-5) of the above addition methods, it is preferable to adjust the addition rate by using an auto feeder or the like. By this, adhesion of the raw material powder to the wall of the stirring container and aggregation of the raw material powders are suppressed, and predetermined amounts are supported on the carrier.

In the method for producing a catalyst of the present invention, at least a sulfur-containing inorganic compound is added to form a raw material powder by such a molding process. When the tumbling granulation method is selected as the molding method, the above-mentioned (1-1) to (1-5) can be appropriately combined for "molding by adding a sulfur-containing inorganic compound". Among these methods, the method (1-5) is preferable because the shape of the catalyst tends to be uniform.

When the tableting molding method is selected, a method is preferred as a method of "adding and molding a sulfur-containing inorganic compound" in which a sulfur-containing inorganic compound is added to the raw material powder in advance to form a mixture, and then the mixture is molded by tablet molding.

[Firing Process]

In the method for producing a catalyst of the present invention, the catalyst precursor obtained in the above molding process is preferably fired at a temperature condition of preferably 300 °C to 500 °C, more preferably 350 °C to 450 °C for about 1 hour to 16 hours.

As the firing method, the method used in the above heat treatment can be adopted.

As the firing atmosphere in the firing process, an atmosphere having an oxygen concentration of 10% by volume or less is preferable from the viewpoint of improving the performance of the obtained catalyst.

According to the methods described above, a catalyst having high activity and excellent yield of the desired unsaturated carboxylic acid can be obtained.

The obtained catalyst preferably contains molybdenum and vanadium as catalyst component elements, and more preferably copper in addition to the molybdenum and vanadium. The obtained catalyst preferably has a composition represented by the aforementioned general formula (1).

The catalyst component element is contained in the powder, and excludes the carrier and the molding aid contained in the catalyst precursor or the catalyst obtained by the production method of the present invention.

[Application]

By using the catalyst produced by the production method of the present invention as a catalyst for synthesizing unsaturated carboxylic acids, the catalyst performance such as raw material conversion rate and product selectivity can be further improved, whereby unsaturated aldehydes such as acrolein and methacrolein can be vapor-phase catalytically oxidized with an oxygen-containing gas to obtain corresponding unsaturated carboxylic acids such as acrylic acid and methacrylic acid in a high yield.

[Acrylic Acid Manufacturing Method]

The method for producing acrylic acid of the present invention is a method for producing acrylic acid by vapor-phase catalytic oxidation of acrolein with an oxygen-containing gas in the presence of the catalyst of the present invention.

A type of a reactor used in this method is not particularly limited. As the reactor, a fixed bed reactor, a fluidized bed reactor and the like can be used for example. As the reactor, a fixed bed reactor is preferable, and a fixed bed multi-tube reactor is more preferable.

Vapor-phase catalytic oxidation is performed by contacting a mixed gas containing acrolein and molecular oxygen (hereinafter, may be referred to as "raw material gas") with the catalyst in the reactor.

When acrylic acid is produced by vapor-phase catalytic oxidation of acrolein using the catalyst of the present invention, the concentration of acrolein in the raw material gas can be changed in a wide range. The concentration of acrolein in the raw material gas is preferably 1% by volume to 20% by volume, more preferably 3% by volume to 10% by volume. The raw material containing acrolein may contain a small amount of impurities such as water and lower saturated aldehyde that do not substantially affect the reaction.

The amount of molecular oxygen in the raw material gas is preferably 0.4 mol to 4 mol times, more preferably 0.5 mol to 3 mol times of acrolein. It is industrially advantageous to use air as the molecular oxygen source of the raw material gas. As the molecular oxygen source, air enriched with pure oxygen can also be used, if necessary. The raw material gas is preferably diluted with an inert gas such as nitrogen and carbon dioxide, and steam.

The reaction pressure in the vapor-phase catalytic oxidation reaction is preferably about atmospheric pressure to several atmospheric pressure. The reaction is preferably carried out using a fixed bed. The reaction temperature is preferably 200 °C to 450 °C, more preferably 250 °C to 400 °C. The contact time between the raw material gas and the catalyst is preferably 1.5 seconds to 15 seconds, more preferably 2 seconds to 7 seconds.

EXAMPLES

The present invention will be described in more detail below with reference to Examples. The present invention is not limited to the following examples insofar as not departing from the gist of the present invention.

[Examples 1 to 5, Comparative Examples 1 and 2]

<Catalyst Preparation>

76 g of ammonium metavanadate was added to 2281 mL of warm water and dissolved. Then, 568 g of ammonium molybdate was further added and dissolved to obtain a solution (hereinafter referred to as "solution A").

Solution B, in which 80 g of copper sulfate was added to 115 mL of warm water and dissolved, was added to the solution A and mixed so as to be uniform. Further, 52 g of niobium hydroxide and 16 g of antimony trioxide were further added thereto and mixed to obtain a starting material mixture.

The starting material mixture was spray-dried at 150 °C. Then, the spray-dried product was heat-treated in the air at a heating temperature of 300 °C for 1 hour to obtain a dried product.

This dried product was pulverized to 200 $\mu$m or less using a stirring blade type crusher to obtain a pulverized product.

This pulverized product is the raw material powder used for molding.

1.5 parts by weight of scaly glass was added to 100 parts by weight of the raw material powder and mixed so as to be uniform to obtain a mixture.

100 g of a spherical inert carrier having a diameter of 4.9 mm and containing alumina-silica as a main component was supplied into a pan-type granulator. Then, the mixture and a molding aid shown in Table 1 were alternately added to the inert carrier at the addition amounts shown in Table 1 to be supported on the carrier so that the support ratio was 40% by weight. As a result, a catalyst precursor being a granulated molded product was obtained.

The catalyst precursor was fired at 390 °C for 3 hours in an atmosphere of 2% by volume of oxygen which was diluted air diluted with nitrogen to obtain a catalyst. The composition ratio of the catalyst was as follows.

$$Mo_{12}V_{2.4}Cu_{1.2}Nb_1Sb_{0.4}$$

<Vapor-Phase Catalytic Oxidation Reaction of Acrolein>

Using the catalysts obtained in each Example and Comparative Example, a vapor-phase catalytic oxidation reaction of acrolein was carried out under the following conditions, and the acrolein conversion rate, acrylic acid selectivity and acrylic acid yield were examined. The results are shown in Table 1.

A reaction tube (inner diameter 21 mm) having a jacket holding niter as a heat medium therein was packed with 33 mL of the catalyst. The vapor-phase catalytic oxidation reaction of acrolein was carried out by heating the reaction tube and introducing a raw material gas (acrolein 6% by volume, oxygen 8% by volume, steam 22% by volume, nitrogen 64% by volume) with an SV (space velocity; flow rate of the raw material gas per unit time / apparent volume of the catalyst packed therein) of 1550 / hr. The heat medium temperature was 250 °C.

The acrolein conversion rate, acrylic acid selectivity, and acrylic acid yield are defined as the following formulas (1) to (3).

(1) Acrolein conversion rate (mol%) = 100 * (number of moles of reacted acrolein) / (number of moles of supplied acrolein)

(2) Acrylic acid selectivity (mol%) = 100 * (number of moles of produced acrylic acid) / (number of moles of converted acrolein)

(3) Acrylic acid yield (mol%) = 100 * (number of moles of produced acrylic acid) / (number of moles of supplied acrolein)

[Table 1]

| | Molding Aid | | | | Reaction Result | | |
|---|---|---|---|---|---|---|---|
| | Ammonium Sulphate or Ammonium Nitrate Addition Amount *1 (weight part) | Ammonium Sulphate or Ammonium Nitrate Addition Form | Glycerin Addition Amount *2 (weight part) | Glycerin Addition Form | Acrolein Conversion Rate (mol%) | Acrylic Acid Selectivity (mole%) | Acrylic Acid Yield (mole%) |
| Example 1 | 7.30 | Ammonium Sulphate 30% by weight Aqueous Solution | 0 | - | 98.3 | 96.4 | 94.9 |
| Example 2 | 7.70 | Ammonium Sulphate 30% by weight Aqueous Solution | 2.57 | 10% by weight Aqueous Solution | 99.9 | 95.8 | 95.8 |
| Example 3 | 7.60 | Ammonium Sulphate 30% by weight Aqueous Solution | 1.27 | 5% by weight Aqueous Solution | 99.9 | 95.6 | 95.6 |
| Example 4 | 4.91 | Ammonium Sulphate 20% by weight Aqueous Solution | 1.23 | 5% by weight Aqueous Solution | 99.9 | 95.2 | 95.2 |

(continued)

| | Molding Aid | | | | Reaction Result | | |
|---|---|---|---|---|---|---|---|
| | Ammonium Sulphate or Ammonium Nitrate Addition Amount *1 (weight part) | Ammonium Sulphate or Ammonium Nitrate Addition Form | Glycerin Addition Amount *2 (weight part) | Glycerin Addition Form | Acrolein Conversion Rate (mol%) | Acrylic Acid Selectivity (mole%) | Acrylic Acid Yield (mole%) |
| Example 5 | 2.27 | Ammonium Sulphate 10% by weight Aqueous Solution | 1.14 | 5% by weight Aqueous Solution | 99.9 | 95.1 | 95.0 |
| Comparative Example 1 | 0 | - | 2.14 | 10% by weight Aqueous Solution | 99.7 | 95.0 | 94.7 |
| Comparative Example 2 | 8.19 | Ammonium Nitrate 30% by weight Aqueous Solution | 0 | - | 81.9 | 96.5 | 79.0 |

*1: Ammonium sulfate or ammonium nitrate addition amount (parts by weight): Addition amount to 100 parts by weight of raw material powder *2: Amount of glycerin added (parts by weight) Amount added to 100 parts by weight of raw material powder

As described above, it is possible to produce a catalyst having a high raw material conversion rate and a high product selectivity as well as an excellent yield of acrylic acid by molding a raw material powder using a sulfur-containing inorganic compound according to the present invention.

While the present invention has been described in detail with reference to the specific embodiments, it will be apparent to those skilled in the art that the present invention can be variously modified in practical use without departing from the spirit and the scope of the present invention.

This application is based on Japanese Patent Application No. 2020-14788 filed on January 31, 2020, the entire contents of which are incorporated herein by reference.

**Claims**

1. A method for producing a catalyst for synthesizing an unsaturated carboxylic acid, the method comprising a molding process of molding a powder containing each catalyst component element to produce a catalyst precursor, wherein a sulfur-containing inorganic compound is added to the powder, and the powder is molded in the molding process.

2. The method for producing a catalyst according to claim 1, wherein the sulfur-containing inorganic compound does not contain the catalyst component element.

3. The method for producing a catalyst according to claim 1 or 2, wherein the sulfur-containing inorganic compound is a sulfate.

4. The method for producing a catalyst according to claim 3, wherein the sulfate is ammonium sulfate.

5. The method for producing a catalyst according to any one of claims 1 to 4, wherein an amount of the sulfur-containing inorganic compound added is 1.0 part by weight or more and 15 parts by weight or less with respect to 100 parts by weight of the powder.

6. The method for producing a catalyst according to any one of claims 1 to 5, wherein an organic compound is further added to the powder, and the powder is molded in the molding process.

7. The method for producing a catalyst according to claim 6, wherein the organic compound is glycerin.

8. The method for producing a catalyst according to claim 6 or 7, wherein an amount of the organic compound added is 0.3 parts by weight or more and 15 parts by weight or less with respect to 100 parts by weight of the powder.

9. The method for producing a catalyst according to any one of claims 1 to 8, wherein the catalyst component element contains molybdenum and vanadium.

10. The method for producing a catalyst according to any one of claims 1 to 9, wherein the molding process is a process of supporting the powder on a surface of a carrier and granulating the powder into a catalyst precursor.

11. A method for producing acrylic acid in which acrolein is oxidized by a vapor-phase catalytic oxidization reaction with an oxygen-containing gas in the presence of a catalyst produced by the method for producing a catalyst according to any one of claims 1 to 10.

## INTERNATIONAL SEARCH REPORT

International application No.

PCT/JP2020/037422

### A. CLASSIFICATION OF SUBJECT MATTER
B01J 37/00(2006.01)i; B01J 23/887(2006.01)i; C07B 61/00(2006.01)i; C07C 51/235(2006.01)i; C07C 57/055(2006.01)i
FI: B01J37/00 E; C07C51/235; C07B61/00 300; B01J23/887 Z; C07C57/055 A
According to International Patent Classification (IPC) or to both national classification and IPC

### B. FIELDS SEARCHED

Minimum documentation searched (classification system followed by classification symbols)
B01J21/00-38/74; C07B61/00; C07C51/235; C07C57/055

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 55-79340 A (UBE INDUSTRIES, LTD.) 14 June 1980 (1980-06-14) page 1, upper right column, lines 3-7, page 2, lower right column, line 20 to page 3, lower right column, line 18, page 4, upper left column, lines 4-9, examples 1, 11, 14, 30, tables 2, 3 | 1-6, 8, 9<br>7-11 |
| Y | JP 2016-536136 A (BASF SE) 24 November 2016 (2016-11-24) paragraphs [0045]-[0058], [0097]-[0098], [0108] | 7-11 |
| Y | JP 54-144311 A (ZEON CORP.) 10 November 1979 (1979-11-10) page 2, upper right column, line 18 to lower right column, line 9, page 3, upper left column, lines 5-13, examples 1, 2, 5 | 11 |

☒ Further documents are listed in the continuation of Box C.    ☒ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 November 2020 (16.11.2020) | 01 December 2020 (01.12.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

13

**INTERNATIONAL SEARCH REPORT**

International application No.

PCT/JP2020/037422

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2011-527243 A (BASF SE) 27 October 2011 (2011-10-27) claims 1, 52, 66 | 1-11 |
| A | JP 2013-202564 A (MITSUBISHI CHEMICAL CORP.) 07 October 2013 (2013-10-07) claims 3, 4, paragraph [0029] | 1-11 |

Form PCT/ISA/210 (continuation of second sheet) (January 2015)

International application No.

PCT/JP2020/037422

| Patent Documents referred in the Report | Publication Date | Patent Family | Publication Date |
|---|---|---|---|
| JP 55-79340 A | 14 Jun. 1980 | (Family: none) | |
| JP 2016-536136 A | 24 Nov. 2016 | US 2015/0080605 A1 paragraphs [0066]-[0079], [0131]-[0132], [0142] WO 2015/039982 A1 EP 3046668 A1 KR 10-2016-0056893 A CN 106687212 A | |
| JP 54-144311 A | 10 Nov. 1979 | (Family: none) | |
| JP 2011-527243 A | 27 Oct. 2011 | US 2010/0010238 A1 claims 1, 52, 66 WO 2010/000764 A2 EP 2307192 A2 KR 10-2011-0026514 A CN 102137750 A | |
| JP 2013-202564 A | 07 Oct. 2013 | (Family: none) | |

Form PCT/ISA/210 (patent family annex) (January 2015)

**EP 4 098 361 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015096497 A
- JP 2020014788 A